(19) Europäisches Patentamt
European Patent Office
Office européen des brevets

(11) Veröffentlichungsnummer: **0 395 985 A2**

(12) **EUROPÄISCHE PATENTANMELDUNG**

(21) Anmeldenummer: **90107708.1**

(22) Anmeldetag: **24.04.90**

(51) Int. Cl.[5]: **C07D 403/12, A01N 43/58, C07D 413/12, C07D 417/12, A01N 43/707, A01N 43/82**

(30) Priorität: **29.04.89 DE 3914337**

(43) Veröffentlichungstag der Anmeldung: **07.11.90 Patentblatt 90/45**

(84) Benannte Vertragsstaaten: **AT BE CH DE DK ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Leyendecker, Joachim, Dr.**
**Stahlbuehlring 79**
**D-6802 Ladenburg(DE)**
Erfinder: **Neubauer, Hans-Juergen, Dr.**
**Mozartstrasse 6**
**D-4400 Muenster-Hiltrup(DE)**
Erfinder: **Kardorff, Uwe, Dr.**
**D 3, 4**
**D-6800 Mannheim 1(DE)**
Erfinder: **Kuekenhoehner, Thomas, Dr.**
**Seidelstrasse 2**
**D-6710 Frankenthal(DE)**
Erfinder: **Kuenast, Christoph, Dr.**
**Salierstrasse 2**
**D-6701 Otterstadt(DE)**
Erfinder: **Hofmeister, Peter, Dr.**
**Bernard-Humblot-Strasse 12**
**D-6730 Neustadt(DE)**

(54) **3(2H)-Pyridazinonderivate, Verfahren zu ihrer Herstellung und ihre Verwendung zur Bekämpfung von Schädlingen.**

(57) 3(2H)-Pyridazinonderivate der allgemeinen Formel I

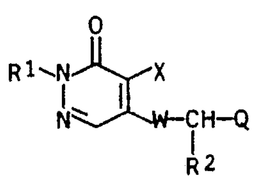

(I)

in der die Substituenten die folgende Bedeutung haben:
$R^1$ $C_1$-$C_8$-Alkyl,
$R^2$ Wasserstoff oder $C_1$-$C_4$-Alkyl,
X Halogen,
W Sauerstoff oder Schwefel und
Q einen gegebenenfalls substituierten 5- oder 6-gliedrigen Heterocyclus mit 2 bis 4 Heteroatomen aus der Gruppe Stickstoff, Sauerstoff oder Schwefel,
mit der Maßgabe, daß Q keinen Isoxazolyl- oder Pyridazinylrest bedeutet,
sowie Verfahren zur Herstellung der Verbindungen I und ihre Verwendung als Schädlingsbekämpfungsmittel.

EP 0 395 985 A2

# 3(2H)-Pyridazinonderivate, Verfahren zu ihrer Herstellung und ihre Verwendung zur Bekämpfung von Schädlingen

Die vorliegende Erfindung betrifft neue 3(2H)-Pyridazinonderivate der allgemeinen Formel I

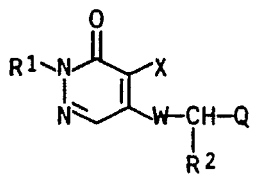

(I)

in der die Substituenten die folgende Bedeutung haben:
$R^1$ $C_1$-$C_8$-Alkyl,
$R^2$ Wasserstoff oder $C_1$-$C_4$-Alkyl,
X Halogen,
W Sauerstoff oder Schwefel und
Q einen 5- oder 6-gliedrigen Heterocyclus mit 2 bis 4 Heteroatomen aus der Gruppe Stickstoff, Sauerstoff oder Schwefel, der unsubstituiert oder ein- bis dreifach substiuiert ist mit Halogen, $C_1$-$C_8$-Alkyl, $C_2$-$C_6$-Alkenyl, $C_2$-$C_4$-Alkinyl, $C_1$-$C_4$-Halogenalkyl, $C_1$-$C_8$-Alkoxy, $C_2$-$C_8$-Alkoxyalkyl, $C_3$-$C_8$-Cycloalkyl, Cyano, Nitro, Aryl und $C_7$-$C_{20}$-Aralkyl, welche unsubstiuiert, oder ein- bis dreifach durch Halogen, $C_1$-$C_8$-Alkyl, $C_1$-$C_8$-Alkoxy, $C_1$-$C_4$-Halogenalkyl, $C_1$-$C_4$-Halogenalkoxy, Cyano oder Nitro substituiert sind,
mit der Maßgabe, daß Q keinen Isoxazolyl- und Pyridazinylrest bedeutet,
sowie pflanzenverträgliche Salze der 3(2H)-Pyridazinonderivate I.

Außerdem betrifft die Erfindung die Herstellung der Verbindungen I, Schädlingsbekämpfungsmittel, die die Verbindungen I als Wirkstoffe enthalten sowie ein Verfahren zur Bekämpfung von Schädlingen.

In den älteren deutschen Patentanmeldungen P 37 42 266.9 (EP-A-320 733) und P 38 44 227.2 sind 5-Isoxazolylmethyl-3(2H)-pyridazinone beschrieben. Weiterhin sind aus EP-A-199 281 zahlreiche hetarylisch substituierte 3(2H)-Pyridazinone bekannt, die als Hetarylreste aber nur Pyrrol, Furan, Thiophen, Pyridin und Pyridazin enthalten. In der Patentanmeldung EP-A-302 346 sind ebenfalls zahlreiche hetarylisch substituierte 3(2H)-Pyridazinone beschrieben, die aber als Rest $R^1$ keinen $C_1$-$C_8$-Alkylrest enthalten.

Da die insektizide und akarizide Wirkung der vorbeschriebenen Verbindungen nicht immer befriedigend ist, lag der Erfindung die Aufgabe zugrunde, neue hetarylisch substituierte 3(2H)-Pyridazinonderivate mit verbesserter Wirkung bereitzustellen.

Demgemäß wurden die eingangs definierten neuen 3(2H)-Pyridazinonderivate der allgemeinen Formel I, sowie ein Verfahren zu ihrer Herstellung gefunden. Ferner wurde gefunden, daß sich die Verbindungen I bei sehr guter Pflanzenverträglichkeit hervorragend zur Bekämpfung von Schädlingen eignen.

Die Substituenten in Formel I haben im einzelnen folgende Bedeutungen:
$R^1$ unverzweigtes oder verzweigtes $C_1$-$C_8$-Alkyl, bevorzugt $C_1$-$C_6$-Alkyl, besonders bevorzugt $C_1$-$C_4$-Alkyl wie Methyl, Ethyl, n-Propyl, iso-Propyl, n-Butyl, iso-Butyl, sec.-Butyl und tert.-Butyl,
$R^2$ Wasserstoff, unverzweigtes oder verzweigtes $C_1$-$C_4$-Alkyl wie für $R^1$ aufgeführt, bevorzugt $C_1$-$C_2$-Alkyl, besonders bevorzugt Methyl,
X Halogen, bevorzugt Chlor oder Brom, besonders bevorzugt Chlor,
W Sauerstoff oder Schwefel, besonders bevorzugt Schwefel,
Q einen 5- oder 6-gliedrigen Heterocyclus mit 2 bis 4, insbesondere 2 bis 3 Heteroatomen wie Stickstoff, Sauerstoff oder Schwefel, der unsubstituiert oder ein bis dreifach substituiert ist, wobei bevorzugt Pyrazol, Imidazol, 1,2,4-Triazol, Isothiazol, Oxazol, Thiazol, 1,3,4-Oxadiazol, 1,3,4-Thiadiazol, 1,2,4-Oxadiazol, Pyrimidin, Pyrazin, 1,3,5-Triazin, 1,2,3-Triazin und 1,2,4-Triazin, besonders bevorzugt Pyrazol, Imidazol, 1,2,4-Triazol, Isothiazol, Oxazol, Thiazol, 1,3,4-Oxadiazol, 1,3,4-Thiadiazol, 1,2,4-Oxadiazol, 1,2,4-Thiadiazol, Pyrimidin und Pyrazin zu nennen sind und folgende Substituenten in Betracht kommen:
Halogen bevorzugt Fluor, Chlor und Brom, besonders bevorzugt Fluor und Chlor,
$C_1$-$C_8$-Alkyl, bevorzugt $C_1$-$C_4$-Alkyl, besonders bevorzugt Methyl, Ethyl, iso-Propyl, tert.-Butyl,
$C_2$-$C_8$-Alkenyl, bevorzugt $C_2$-$C_4$-Alkenyl, besonders bevorzugt Ethenyl, 1-Methyl-ethenyl, Propenyl, 2-Methyl-propenyl,
$C_1$-$C_4$-Halogenalkyl, bevorzugt mit Fluor und/oder Chlor substituiertes $C_1$-$C_2$-Halogenalkyl, besonders bevorzugt Trifluormethyl, 2,2,2-Trifluorethyl, Trichlormethyl,
$C_1$-$C_8$-Alkoxy, bevorzugt $C_1$-$C_3$-Alkoxy, besonders bevorzugt Methoxy, Ethoxy, n-Propyloxy und iso-Propy-

loxy,
$C_2$-$C_8$-Alkoxyalkyl, bevorzugt $C_2$-$C_4$-Alkoxyalkyl, besonders bevorzugt Methoxymethyl, 1-Methoxyethyl, 2-Methoxyethyl, 1-Methoxy-propyl,
$C_3$-$C_8$-Cycloalkyl, bevorzugt $C_3$-$C_5$-Cycloalkyl wie Cyclopropyl, Cyclobutyl und Cyclopentyl,
Cyano,
Nitro,
Aryl, bevorzugt Phenyl und Naphthyl, besonders bevorzugt Phenyl,
$C_7$-$C_{20}$-Aralkyl, bevorzugt $C_7$-$C_{12}$-Phenylalkyl, besonders bevorzugt Benzyl und Phenethyl.

Der Arylrest und der $C_7$-$C_{20}$-Aralkylrest sind unsubstituiert oder ein bis dreifach substituiert mit:
Halogen, bevorzugt Fluor, Chlor und Brom, besonders bevorzugt Fluor und Chlor,
$C_1$-$C_8$-Alkyl, bevorzugt $C_1$-$C_6$-Alkyl, besonders bevorzugt $C_1$-$C_4$-Alkyl, wie Methyl, Ethyl, n-Propyl, iso-Propyl, n-Butyl, iso-Butyl, sec.-Butyl und tert.-Butyl,
$C_1$-$C_8$-Alkoxy, bevorzugt $C_1$-$C_6$-Alkoxy, besonders bevorzugt $C_1$-$C_4$-Alkoxy, wie Methoxy, Ethoxy, n-Propyloxy, iso-Propyloxy, n-Butyloxy, iso-Butyloxy, sec.-Butyloxy und tert.-Butyloxy,
$C_1$-$C_4$-Halogenalkyl, bevorzugt $C_1$-$C_3$-Fluor- und/oder Chloralkyl, besonders bevorzugt $C_1$-$C_2$-Fluor- und/oder Chloralkyl, wie Fluormethyl, Difluormethyl, Trifluormethyl, Chlormethyl, Dichlormethyl, Trichlormethyl, Chlorfluormethyl, Chlordifluormethyl, Dichlorfluormethyl, 1,1,2,2-Tetrafluorethyl, 1,2,2,2-Tetrafluorethyl, 1,1,2,2-Tetrachlorethyl und 1,2,2,2-Tetrachlorethyl,
$C_1$-$C_4$-Halogenalkoxy, bevorzugt $C_1$-$C_3$-Fluor- und/oder Chloralkoxy, besonders bevorzugt $C_1$-$C_2$-Fluor- und/oder Chloralkoxy, wie Fluormethoxy, Difluormethoxy, Trifluormethoxy, Chlormethoxy, Dichlormethoxy, Trichlormethoxy, Chlorfluormethoxy, Chlordifluormethoxy, Dichlorfluormethoxy, 1,1,2,2-Tetrafluorethoxy, 1,2,2,2-Tetrafluorethoxy, 1,1,2,2-Tetrachloroethoxy, 1,2,2,2-Tetrachlorethoxy,
Cyano und
Nitro.

Die Verbindungen I sind nach folgender Methode erhältlich:

Die Umsetzung erfolgt zwischen einem 3(2H)-Pyridazinon der allgemeinen Formel II und einer Verbindung der allgemeinen Formel III in Gegenwart einer Base im Temperaturbereich von (-20) bis 250°C, vorzugsweise von 20 bis 120°C nach folgender Reaktionsgleichung:

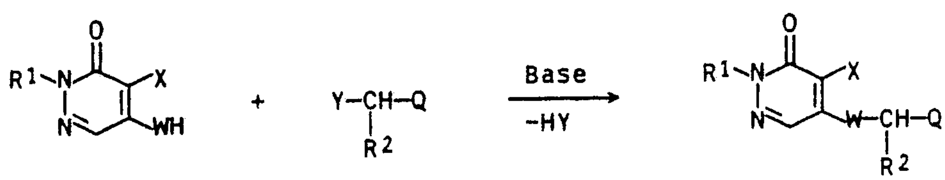


Die 3(2H)-Pyridazinone der allgemeinen Formel II sind zum Teil in dem belgischen Patent 607 934; in EP-A-134 439, in Angew. Chem. 72, 864 ff (1960) und Chem. Pharm. Bull. 18, 147 ff (1970) beschrieben oder können nach den dort beschriebenen Methoden hergestellt werden.

Die Heterocyclen sind entweder bekannt und teilweise kommerziell erhältlich oder lassen sich nach bekannten chemischen Verfahren herstellen. Verfahren zur Herstellung von Pyrazolen finden sich beispielsweise in: Heterocyclic Nitrogen Compounds, The Azoles, S. 31 ff, Cambridge University Press, 1976; Verfahren zur Herstellung von Imidazolen beispielsweise in: Advances in Heterocyclic Chem., Vol. 27, S. 242 ff, 1980; Verfahren zur Herstellung von Triazolen beispielsweise in: Comprehensive Heterocyclic Chemistry, A.R. Katritzky und C.W. Rees, Vol. 5, S. 669 ff, Pergamon Press, 1984; Verfahren zur Herstellung von Isothiazolen, Oxazolen, Thiazolen, Oxadiazolen und Thiadiazolen beispielsweise in: Comprehensive Heterocyclic Chemistry, A.R. Katritzky und C.W. Rees, Vol. 6, S. 131, 177, 235, 365, 447 ff, Pergamon Press, 1984.

Der Rest Y bedeutet eine Abgangsgruppe wie beispielsweise einen Sulfonsäurerest oder ein Halogen. Unter den Sulfonsäureresten sind Methansulfonyl, Trifluormethansulfonyl, Benzolsulfonyl und p-Toluolsulfonyl, unter den Halogenen sind Chlor und Brom bevorzugt, besonders bevorzugt wird Chlor.

Zur Herstellung der erfindungsgemäßen Verbindungen I nach der vorstehend beschriebenen Methode setzt man die Ausgangsstoffe üblicherweise in stöchiometrischem Verhältnis ein. Ein Überschuß der einen oder anderen Komponente kann von Vorteil sein.

Die Umsetzungen verlaufen gewöhnlich oberhalb von -20°C mit ausreichenden Geschwindigkeiten. 120°C müssen im allgemeinen nicht überschritten werden. Da sie in einigen Fällen unter Wärmeentwicklung verlaufen, kann es von Vorteil sein, Kühlmöglichkeiten vorzusehen.

Man setzt normalerweise mindestens äquivalente Mengen einer Base zu II und/oder III zu, kann aber

diese auch im Überschuß oder gegebenenfalls auch als Lösungsmittel verwenden. Als Basen eignen sich beispielsweise Hydroxide von Alkali- und Erdalkalimetallen, wie Natriumhydroxid, Kaliumhydroxid und Calciumhydroxid, Alkoholate von Alkali- und Erdalkalimetallen, wie Natriummethylat, Natriumethylat, Calciummethanolat oder Kalium-tert.-butylat; Alkali- oder Erdalkalimetallhydride, wie Natriumhydrid, Kaliumhydrid oder Calciumhydrid; Alkali- oder Erdalkalicarbonate, wie Natriumcarbonat, Kaliumcarbonat oder Calciumcarbonat; aliphatische Amine, wie Dimethylamin, Triethylamin oder Diisopropylamin; heterocyclische Amine, wie Piperidin, Piperazin oder Pyrrolidin; aromatische Amine, wie Pyridin oder Pyrrol.

Die Umsetzung wird zweckmäßigerweise in einem Lösungs- oder Verdünnungsmittel durchgeführt. Hierzu eignen sich bespielsweise aliphatische Kohlenwasserstoffe, wie n-Pentan, n-Hexan, das Hexan-Isomerengemisch und Petrolether; halogenierte Kohlenwasserstoffe wie Chlorbenzol, Methylenchlorid, Ethylenchlorid, Chloroform, Tetrachlorethylen; aromatische Kohlenwasserstoffe, wie Benzol, Toluol, die Xylole und deren Isomerengemische, Benzin; Alkohole, wie Methanol, Ethanol, n-Propanol und Isopropanol; Ether, wie Diethyl-, Di-n-butylether, Methyl-tert.-butylether, Tetrahydrofuran und Dioxan; Ketone, wie Aceton, Methylethylketon und Methylisopropylketon; Nitrile, wie Acetonitril und Propionitril; aprotische dipolare Lösungsmittel wie Dimethylformamid, Dimethylsulfoxid oder Pyridin. Auch Gemische dieser Stoffe können als Lösungs- und Verdünnungsmittel verwendet werden.

Zweckmäßigerweise wird das 3(2H)-Pyridazinon der allgemeinen Formel II in einem Verdünnungs- oder Lösungsmittel vorgelegt, unter anschließender zugabe des Ausgangsstoffes III. Zur Isolierung der neuen Verbindungen wird nach üblichen Methoden vorgegangen. Die anfallenden Produkte können durch Umkristallisieren, Extrahieren oder Chromatographieren gereinigt werden.

Zur Darstellung von pflanzenverträglichen Salzen können die entsprechenden 3(2H)-Pyridazinone I mit üblicherweise verwendeten Salzbildnern, wie z.B. Chlorwasserstoffsäure, Bromwasserstoffsäure, Jodwasserstoffsäure, Schwefelsäure, Phosphorsäure, Ameisensäure, Essigsäure, Oxalsäure, Benzolsulfonsäure, p-Toluolsulfonsäure, Dodecylbenzolsulfonsäure, Methylchlorid, Methylbromid, Methyljodid, Ethylchlorid, Ethylbromid, Ethyljodid, n-Propylchlorid, n-Propylbromid, Dimethylsulfat und Diethylsulfat im Temperaturbereich von 0 bis 150 °C, vorzugsweise von 20 bis 120 °C umgesetzt werden.

Die Umsetzung wird zweckmäßigerweise in einem Lösungs- oder Verdünnungsmittel durchgeführt. Hierzu eignen sich beispielsweise aliphatische Kohlenwasserstoffe wie n-Pentan, n-Hexan, das Hexan-Isomerengemisch und Petrolether; aromatische Kohlenwasserstoffe wie Benzol, Toluol, die Xylole und deren Isomerengemische, Benzin; Ether wie Diethylether, Di-n-butylether, Methyl-tert.-butylether, Tetrahydrofuran und Dioxan; Ketone wie Aceton, Methylethylketon und Methylisopropylketon; halogenierte Kohlenwasserstoffe wie Chlorbenzol, Methylenchlorid, Ethylenchlorid, Chloroform und Tetrachlorethylen. Auch Gemische dieser Stoffe können als Lösungsmittel verwendet werden.

Zur Herstellung der Salze geeigneter Verbindungen I nach der vorstehend beschriebenen Methode setzt man die Ausgangsstoffe üblicherweise in stöchiometrischem Verhältnis ein. Ein Überschuß der einen oder anderen Komponente kann aber durchaus von Vorteil sein.

Die Umsetzungen verlaufen gewöhnlich oberhalb von 0 °C mit ausreichenden Geschwindigkeiten. 120 °C müssen im allgemeinen nicht überschritten werden. Da sie in einigen Fällen unter Wärmeentwicklung verlaufen, kann es von Vorteil sein, Kühlmöglichkeiten vorzusehen.

Zur Isolierung der Salze der erfindungsgemäßen Verbindungen I wird nach üblichen Methoden vorgegangen. Die anfallenden Produkte können durch Umkristallisieren, Extrahieren oder Chromatographieren gereinigt werden.

Die 3(2H)-Pyridazinonderivate der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana,

Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Ortiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Geradflügler (Orthoptera) beispielsweise Forficula auricularia, Acheta domestica, Gryllotalpa gryllotalpa, tachycines asynamorus, Locusta migratoria, Stauronotus meroccanus, Schistocerca peregrina, Nomadacris septemfasciata, Melanoplus spretus, Melanoplus femur-rubrum, Blatta orientalis, Blattella germanica, Periplaneta americana, Blabera gigantes.

Zur Klasse der Arachnoidea gehören Spinnentiere (Acarina) beispielsweise Ixodes ficinus, Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Paratylenchus neglectus, Paratylenchus penetrans, Paratylenchus curvitatus, Paratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall eine möglichst feine Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Fettalkoholsulfate, fettsaure Alkali-und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 10 werden mit 95 Gew.-Teilen feinteiligem Kaolin innig vermischt. Man erhält auf diese Weise ein Stäubemittel, das 5 Gew.-% des Wirkstoffs enthält.

II. 30 Gew.-Teile der Verbindung Nr. 50 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 83 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 10 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 50 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,02 bis 10, vorzugsweise 0,1 bis 2,0 kg/ha.

Die erfindungsgemäßen Mittel besitzen ebenfalls ausgeprägte molluskizide Eigenschaften sowohl bei Nackt- als auch bei Gehäuseschnecken und eignen sich hervorragend zur Schneckenbekämpfung in landwirtschaftlichen und gärtnerischen Pflanzenkulturen.

Erfindungsgemäß erhält man ein gegen Schnecken wirksames, z.B. als Streumittel formulierbares Präparat durch Verwendung von 3(2H)-Pyridazinonderivaten I in einer wirksamen Menge.

Geeignete Formulierungen sind z.B. in GB-A-2 087 723 und EP-A-190 595 beschrieben. Sie enthalten im allgemeinen einen Fraßstoff, ein Bindemittel, Konservierungsmittel, Farbstoffe, Lockstoffe, Füllmittel, Repellents, Wasser, organische Solventien, oberflächenaktive Stoffe und den Wirkstoff.

Als Fraßstoffe können alle üblichen in derartigen Ködern verwendbaren Futtersubstanzen enthalten sein. Vorzugsweise in Betracht kommen gemahlenes Getreide, wie z.B. Weizenmehl, Weizenschrot und Gerstenschrot, ferner Sojaschrot, Kleie, Reisstärke, Fischmehl, Fleischmehl und Melasse. Es kann entweder nur ein Fraßstoff oder auch ein Gemisch aus mehreren Fraßstoffen vorhanden sein.

Als Bindemittel können alle üblichen für derartige Zwecke verwendbaren Kleber vorhanden sein. Vorzugsweise infrage kommen Methylcellulose, Zucker, Dextrin, Stärke, Alginate, Glycole, Polyvinylpyrrolidon, Lingninsulfonat, Gummiarabicum, Polyvinylalkohol und Polyvinylacetat. Es können ein oder mehrere Bindemittel enthalten sein.

Als Beispiele für gegebenenfalls vorhandene Konservierungsstoffe seien 2-Hydroxibiphenyl, Sorbinsäure, p-Hydroxybenzaldehyd, p-Hydroxybenzoesäuremethylester, Benzaldehyd, Benzoesäure, p-Hydroxybenzoesäurepropylester und p-Nitrophenol genannt.

Als Farbstoffe, die als Zusatzstoffe in Betracht kommen, seien anorganische Pigmente, wie Eisenoxid, Titandioxid und Ferrocyanblau und organische Farbstoffe, wie Anthrachinon-, Azo- und Metallphthalocyaninfarbstoffe genannt.

Als Stoffe, die einen Lockreiz auf Bodenschädlinge ausüben, können alle üblichen für diesen Zweck geeigneten Komponenten verwendet werden. Beispielhaft genannt seien Anis und Anisöl.

Als Füllmittel kommen alle üblichen für diesen Zweck verwendbaren Stoffe in Betracht. Vorzugsweise genannt seien Kaoline, Tonerden, Talkum, Kreide und Quarzpulver.

Als Repellents, die eine abweisende Wirkung auf warmblütige Lebewesen wie Hund und Igel ausüben, können alle üblichen für diesen Zweck geeigneten Komponenten eingesetzt werden. Beispielhaft genannt sei Nonylsäurevanillylamid.

Als organische Solventien kommen alle üblicherweise für die Herstellung von Ködern verwendbaren organischen Lösungsmittel in Frage. Vorzugsweise in Betracht kommen niedrig siedende organische Solventien, wie Methanol, Ethanol, Butanol und Methylenchlorid.

Als geeignete oberflächenaktive Stoffe kommen in Betracht nicht-ionische Wirkstoffe, wie Kondensationsprodukte von Polyalkylenoxiden und Alkylphenolen und Fettsäurepolyoxyalkylenestern, z.B. Octylphenoxypolyoxyethanol; kationische Wirkstoffe wie quartäre Ammoniumsalze, z.B. Cetyltrimethylammoniumchlorid oder Cetylpyridiniumchlorid und anionische Wirkstoffe, wie die Natriumsalze von langkettigen Alkylsulfaten, z.B. Natriumlaurylsulfat, Salze von Alkylarylsulfaten, das Natriumsalz der Desoxycholsäure, das Natriumsalz der Taurocholsäure, das Natriumsalz der Tauroglykocholsäure.

Eine andere bevorzugte Anwendungsform ist die Saatgutbeizung mit einer für Beizungen üblichen Formulierung.

Der Wirkstoffgehalt in den einzelnen Anwendungsformen kann in weiten Grenzen variieren, z.B. im Bereich von 0,001 bis 90, insbesondere 0,5 bis 50, vorzugsweise 1 bis 10 Gew.% bei Kornformulierungen und 10 bis 90 Gew.% bei Saatgutbeizen.

Die molluskizide Wirkung der erfindungsgemäßen Mittel erstreckt sich auf landbewohnende und amphibische Schnecken, z.B. solche der Gattungen Deroceras (Agriolimax), Limax, Helix, Helicogona, Cepaea, Milax, Lymnaea (Galba), Achatina, Theba, Cochlicella, Helicarion, Vaginulus. Zu den Schadschnekken gehören z.B. die Nacktschnecken (slugs) Arion ater, A. lusitanicus, A. hortenis, Agriolimax reticulatus, Limax flavus, L. maximus, Milax gagates, Mariella dursumierei, Herlicarion salius, Vaginula hedleyi, Pamarion pupillaris sowie die Gehäuseschnecken (snails) Helix aspersa spp., Cepaea nemoralis, Theba pisana, Achatina fulica, Achatina zanzibarica, Bradybaena spp., Cochlodina spp., Helicella spp., Euomphalia spp.

Formulierungsbeispiel VI

In einem Mischer wurden 2 kg Verbindung Nr. 10, 8 kg Calciumstearat, 0,2 kg Natriumbenzoat, 20 kg Kreide, 0,5 kg blauer Farbstoff und 63,3 kg Weizenkleie gemischt. Diese Mischung wurde anschließend in einem Kneter mit ausreichend Wasser befeuchtet und geknetet. Danach wurde die feuchte Mischung in einem Extruder zu Schneckenködergranulat mit einem Durchmesser von 3 mm geformt und bei maximal 60 °C getrocknet.

Formulierungsbeispiel VII

Zur Herstellung einer Saatgutbeize wurden gemischt:
480 g Verbindung Nr. 10
20 g handelsübliches Phenolsulfonsäure-Harnstoff-Formaldehyd-Kondensat
40 g Ethylen-Propylen-Blockcopolymerisat mit einem Molgewicht von 10.000
2 g Xanthan-Gummi
0,5 g Rhodamin FB
80 g 1,2-Propylenglykol
5 g Silikon-Antischaummittel
und mit Wasser auf 1 Liter aufgefüllt.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Herstellungsbeispiele

Beispiel 1

2-tert.-Butyl-4-chlor-5-[(4,5-dichlorimidazol-1-yl)-methylthio]-3(2H)-pyridazinon-3-on (Verbindung Nr. 10)

Zu 8,3 g (0,038 mol) 2-tert.-Butyl-4-chlor-5-mercapto-3(2H)pyridazin-3-on und 5,2 g (0,038 mol) Kaliumcarbonat in 50 ml trockenem Dimethylformamid tropft man bei Raumtemperatur (ca. 20°C) 7 g (0,038 mol) 1-Chlormethyl-4,5-dichlorimidazol in 30 ml trockenem Dimethylformamid. Anschließend rührt man 4 Stunden bei 60°C und über Nacht bei Raumtemperatur (ca. 20°C) nach. Zur Aufarbeitung gießt man in 200 ml Eiswasser und saugt den ausgefallenen Feststoff ab. Nach Umkristallisation aus n-Hexan/Essigester (10/1) erhält man 10,8 g (77,5 % der Theorie) 2-tert.-Butyl-4-chlor-5-[(4,5-dichlorimidazol-1-yl)-methylthio] -3(2H)-pyridazin-3-on als helles Pulver mit Fp.: 161-164°C.

Beispiel 2

2-tert.-Butyl-4-chlor-5-[(5-methyl-1,3,4-thiadiazol-2-yl)-methyl-thio]-3(2H)pyridazin-3-on (Verbindung Nr. 50)

Zu 12 g (0,055 mol) 2-tert.-Butyl-4-chlor-5-mercapto-3(2H)pyridazin-3-on und 7,6 g (0,055 mol) Kaliumcarbonat in 100 ml trockenem Dimethylformamid tropft man bei Raumtemperatur (ca. 20°C) 8,17 g (0,055 mol) 2-Chlormethyl-5-methyl-1,3,4-thiadiazol in 50 ml trockenem Dimethylformamid. Anschließend wird 2 Stunden bei 80°C und über Nacht bei Raumtemperatur (ca. 20°C) nachgerührt. Zur Aufarbeitung gießt man in 300 ml Eiswasser und extrahiert anschließend 3 mal mit je 100 ml Essigester. Die vereinigten Essigesterphasen werden über Magnesiumsulfat getrocknet. Nach dem Verdampfen des Lösungsmittels wird der Rückstand aus n-Hexan/Essigester (6/1) umkristallisiert. Man erhält 8,4 g (46,2 % der Theorie) 2-tert.-Butyl-4-chlor-5[(5-methyl-1,3,4-thiadiazol-2-yl)-methyl-thio]-3(2H )pyridazin-3-on als farbloses Pulver mit Fp.: 163-165°C.

Beispiel 3

2-tert.-Butyl-4-chlor-5-[(3-phenyl-1,2,4-Oxadiazol-5-yl)-methyl-thio]-3(2H)pyridazin-3-on (Verbindung Nr. 83)

Zu 5,1 g (0,023 mol) 2-tert.-Butyl-4-chlor-5-mercapto-3(2H)pyridazin-3-on und 3,17 g (0,023 mol) Kaliumcarbonat in 50 ml trockenem Dimethylformamid tropft man bei Raumtemperatur (ca. 20°C) 4,5 g (0,023 mol) 5-Chlormethyl-3-phenyl-1,2,4-oxadiazol in 30 ml trockenem Diemthylformamid. Anschließend rührt man 2 Stunden bei 80°C und über Nacht bei Raumtemperatur (ca. 20° C) nach. Zur Aufarbeitung gießt man in 200 ml Eiswasser und extrahiert 3 mal mit je 100 ml Essigester. Die organischen Phasen werden

über Magnesiumsulfat getocknet und anschließend das Lösungsmittel im Vakuum verdampft. Nach Umkristallisation aus n-Hexan/Essigester (6/1) erhält man 5,7 g (66 % der Theorie) 2-tert.-Butyl-4-chlor-5-[(3-phenyl-1,2,4-oxadiazol-5-yl)-methyl-thio]-3(2H )pyridazin-3-on als farbloses Pulver mit Fp.: 141-143°C.

Entsprechend dieser Herstellvorschriften können die in der folgenden Tabelle beschriebenen Verbindungen hergestellt werden. Die Substitutionsstellen an den Heterocyclen sind jeweils durch einen Strich gekennzeichnet.

Die ohne Angabe physikalischer Kenngrößen in der nachstehenden Tabelle aufgeführten Verbindungen I können aus den entsprechenden Vorprodukten ohne weiteres erhalten werden und lassen eine gleichartige Wirkung erwarten.

Tabelle: 3(2H)-Pyridazinonderivate I

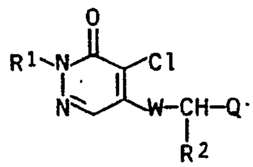

(I)

| Nr. | $R^1$ | $R^2$ | W | Q | Phys. Daten IR-Absorptionen [$cm^{-1}$] oder Fp. [°C] |
|---|---|---|---|---|---|
| 1 | $CH_3$ | H | S | 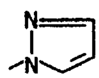 | |
| 2 | i-$C_3H_7$ | H | S | 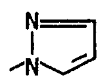 | |
| 3 | t-$C_4H_9$ | H | S | 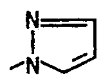 | 126-129 |
| 4 | t-$C_4H_9$ | $CH_3$ | S | 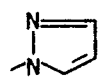 | |
| 5 | $CH_3$ | H | S | 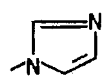 | |
| 6 | t-$C_4H_9$ | H | S | 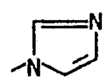 | |
| 7a | $CH_3$ | H | S | 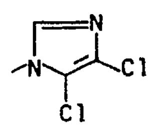 | |
| 7b | $CH_3$ | H | O | 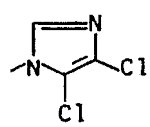 | 142 |
| 8 | i-$C_3H_7$ | H | S | 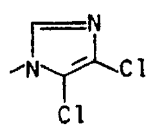 | |
| 9 | t-$C_4H_9$ | H | O | 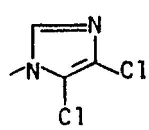 | |
| 10 | t-$C_4H_9$ | H | S | 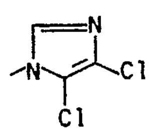 | 161-164 |
| 11 | t-$C_4H_9$ | $CH_3$ | S | 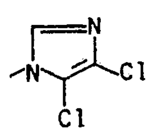 | |

| Nr. | $R^1$ | $R^2$ | W | Q | Phys. Daten IR-Absorptionen [$cm^{-1}$] oder Fp. [°C] |
|---|---|---|---|---|---|
| 12 | $CH_3$ | H | S | 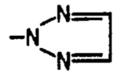 | |
| 13 | i-$C_3H_7$ | H | S | 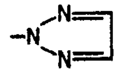 | |
| 14 | t-$C_4H_9$ | H | S | 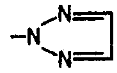 | 115-117 |
| 15 | $CH_3$ | H | S | 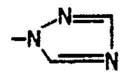 | |
| 16 | $CH_3$ | $CH_3$ | S | 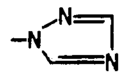 | |
| 17 | i-$C_3H_7$ | H | S | 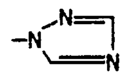 | |
| 18 | t-$C_4H_9$ | H | O | 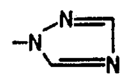 | |
| 19 | t-$C_4H_9$ | $CH_3$ | S | 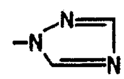 | |
| 20 | t-$C_4H_9$ | H | S | 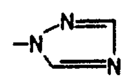 | 84- 88 |
| 21 | t-$C_4H_9$ | H | S | 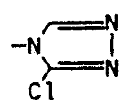 | 130-136 |
| 22 | t-$C_4H_9$ | $CH_3$ | S | 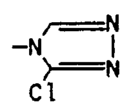 | |
| 23 | t-$C_4H_9$ | H | O | 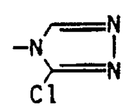 | |
| 24 | i-$C_3H_7$ | H | S | 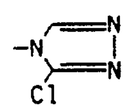 | |
| 25 | $CH_3$ | H | S | 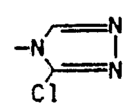 | |
| 26 | t-$C_4H_9$ | H | S | 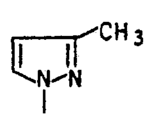 | |

| Nr. | $R^1$ | $R^2$ | W | Q | Phys. Daten IR-Absorptionen [$cm^{-1}$] oder Fp. [°C] |
|---|---|---|---|---|---|
| 27 | $t\text{-}C_4H_9$ | H | S | 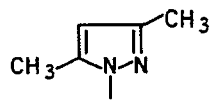 | |
| 28 | $t\text{-}C_4H_9$ | H | S | 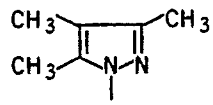 | |
| 29 | $CH_3$ | H | S | 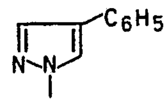 | |
| 30 | $i\text{-}C_3H_7$ | H | S | 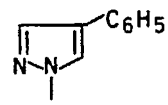 | |
| 31 | $t\text{-}C_4H_9$ | H | S | 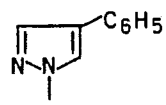 | |
| 32 | $t\text{-}C_4H_9$ | $CH_3$ | S | 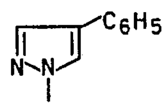 | |
| 33 | $t\text{-}C_4H_9$ | H | S | 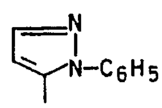 | 86- 89 |
| 34 | $t\text{-}C_4H_9$ | H | S | 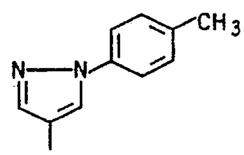 | 111-114 |
| 35 | $t\text{-}C_4H_9$ | H | S | 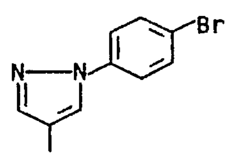 | 163-165 |
| 36 | $CH_3$ | H | S | 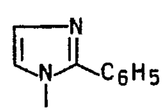 | |
| 37 | $i\text{-}C_3H_7$ | H | S | 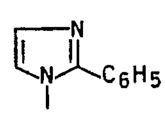 | |
| 38 | $t\text{-}C_4H_9$ | H | S | 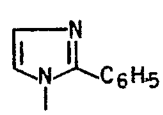 | |

| Nr. | $R^1$ | $R^2$ | W | Q | Phys. Daten IR-Absorptionen [$cm^{-1}$] oder Fp. [°C] |
|---|---|---|---|---|---|
| 39 | $t-C_4H_9$ | H | S | 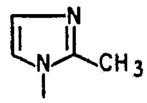 | |
| 40 | $CH_3$ | H | S | 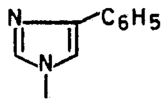 | |
| 41 | $i-C_3H_7$ | H | S | 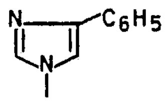 | |
| 42 | $t-C_4H_9$ | H | S | 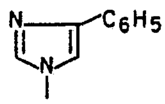 | |
| 43 | $t-C_4H_9$ | $CH_3$ | S | 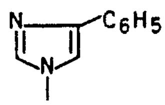 | |
| 44 | $CH_3$ | H | S | 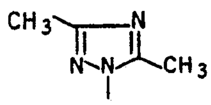 | |
| 45 | $i-C_3H_7$ | H | S | 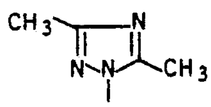 | |
| 46 | $t-C_4H_9$ | H | S | 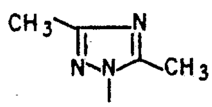 | |
| 47 | $t-C_4H_9$ | $CH_3$ | S | 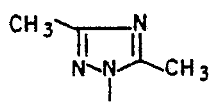 | |
| 48 | $CH_3$ | H | S | 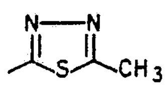 | |
| 49 | $i-C_3H_7$ | H | S | 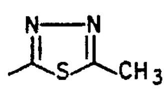 | |
| 50 | $t-C_4H_9$ | H | S | 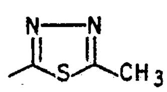 | 163-165 |
| 51 | $t-C_4H_9$ | H | O | 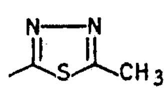 | |
| 52 | $t-C_4H_9$ | H | S | 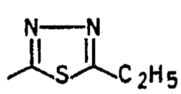 | |

| Nr. | $R^1$ | $R^2$ | W | Q | Phys. Daten IR-Absorptionen [$cm^{-1}$] oder Fp. [°C] |
|---|---|---|---|---|---|
| 53 | t-$C_4H_9$ | H | S | 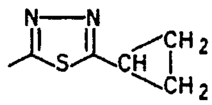 | |
| 54 | $CH_3$ | H | S | 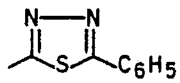 | |
| 55 | i-$C_3H_7$ | H | S | 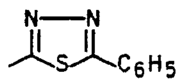 | |
| 56 | t-$C_4H_9$ | H | S | 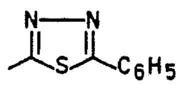 | |
| 57 | t-$C_4H_9$ | $CH_3$ | S | 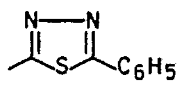 | |
| 58 | $CH_3$ | H | S | 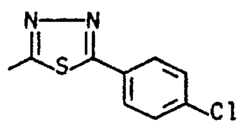 | |
| 59 | i-$C_3H_7$ | H | S | 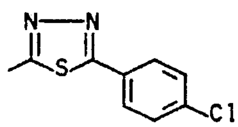 | |
| 60 | t-$C_4H_9$ | H | S | 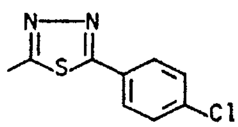 | 161-163 |
| 61 | $CH_3$ | H | S | 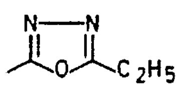 | |
| 62 | i-$C_3H_7$ | H | S | 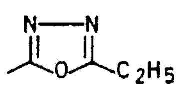 | |
| 63 | t-$C_4H_9$ | H | S | 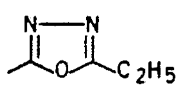 | 123-124 |
| 64 | $CH_3$ | H | S | 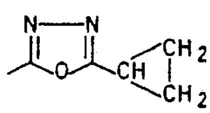 | |
| 65 | i-$C_3H_7$ | H | S | 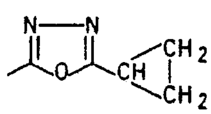 | |
| 66 | t-$C_4H_9$ | H | S | 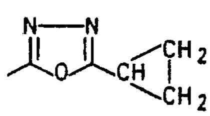 | |

| Nr. | $R^1$ | $R^2$ | W | Q | Phys. Daten IR-Absorptionen [$cm^{-1}$] oder Fp. [°C] |
|---|---|---|---|---|---|
| 67 | $CH_3$ | H | S | 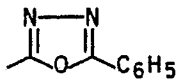 | |
| 68 | i-$C_3H_7$ | H | S | 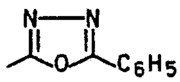 | |
| 69 | t-$C_4H_9$ | H | S | 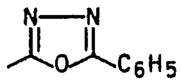 | 163-166 |
| 70 | t-$C_4H_9$ | $CH_3$ | S | 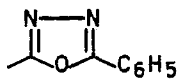 | |
| 71 | t-$C_4H_9$ | H | O | 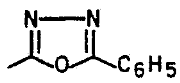 | |
| 72 | $CH_3$ | H | S | 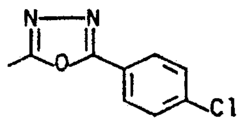 | |
| 73 | i-$C_3H_7$ | H | S | 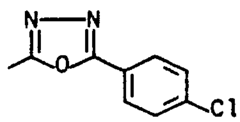 | |
| 74 | t-$C_4H_9$ | H | S | 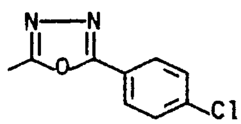 | 128-130 |
| 75 | t-$C_4H_9$ | $CH_3$ | S | 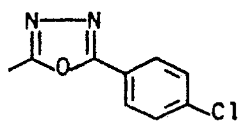 | |
| 76 | $CH_3$ | H | S | 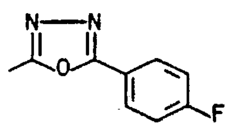 | |
| 77 | i-$C_3H_7$ | H | S | 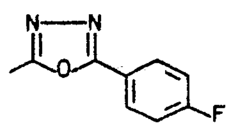 | |
| 78 | t-$C_4H_9$ | H | S | 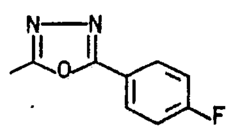 | |
| 79 | t-$C_4H_9$ | $CH_3$ | S | 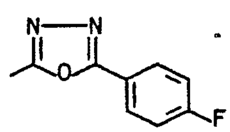 | |

| Nr. | $R^1$ | $R^2$ | W | Q | Phys. Daten IR-Absorptionen [$cm^{-1}$] oder Fp. [°C] |
|---|---|---|---|---|---|
| 80 | t-$C_4H_9$ | H | O | 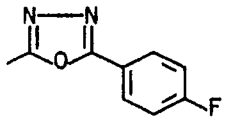 | |
| 81 | $CH_3$ | H | S | 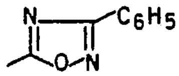 | |
| 82 | i-$C_3H_7$ | H | S | 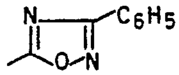 | |
| 83 | t-$C_4H_9$ | H | S | 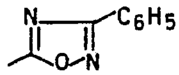 | 141-143 |
| 84 | $CH_3$ | H | S | 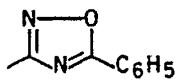 | |
| 85 | i-$C_3H_7$ | H | S | 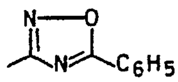 | |
| 86 | t-$C_4H_9$ | H | S | 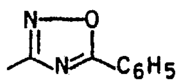 | |
| 87 | t-$C_4H_9$ | $CH_3$ | S | 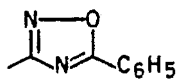 | |
| 88 | t-$C_4H_9$ | H | O | 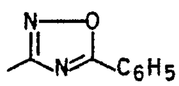 | |
| 89 | $CH_3$ | H | S | 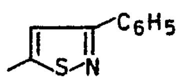 | |
| 90 | i-$C_3H_7$ | H | S | 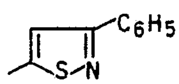 | |
| 91 | t-$C_4H_9$ | H | S | 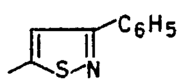 | |
| 92 | t-$C_4H_9$ | $CH_3$ | S | 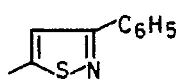 | |
| 93 | t-$C_4H_9$ | H | O | 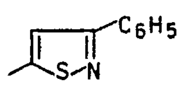 | |
| 94 | $CH_3$ | H | S | 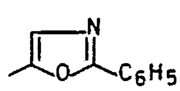 | |
| 95 | i-$C_3H_7$ | H | S | 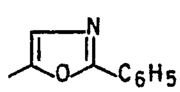 | |

| Nr. | $R^1$ | $R^2$ | W | Q | Phys. Daten IR-Absorptionen [$cm^{-1}$] oder Fp. [°C] |
|---|---|---|---|---|---|
| 96 | $t-C_4H_9$ | H | S | 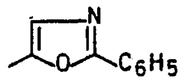 | |
| 97 | $t-C_4H_9$ | $CH_3$ | S | 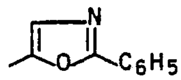 | |
| 98 | $t-C_4H_9$ | H | O | 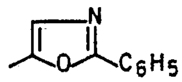 | |
| 99 | $CH_3$ | H | S | 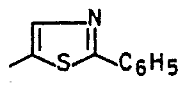 | |
| 100 | $i-C_3H_7$ | H | S | 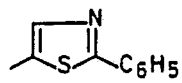 | |
| 101 | $t-C_4H_9$ | H | S | 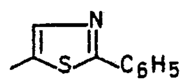 | 1649,1563,1326,1212 |
| 102 | $t-C_4H_9$ | $CH_3$ | S | 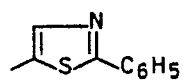 | |
| 103 | $CH_3$ | H | S | 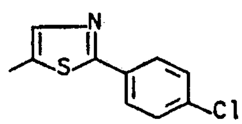 | |
| 104 | $i-C_3H_7$ | H | S | 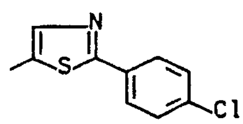 | |
| 105 | $t-C_4H_9$ | H | S | 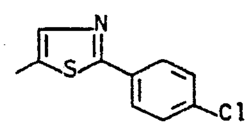 | 1647,1562,1495,1398 |
| 106 | $t-C_4H_9$ | $CH_3$ | S | 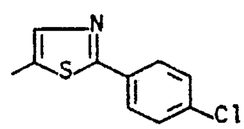 | |
| 107 | $t-C_4H_9$ | H | O | 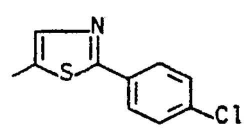 | |
| 108 | $CH_3$ | H | S | 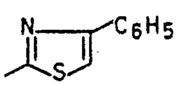 | |
| 109 | $i-C_3H_7$ | H | S | 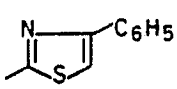 | |

| Nr. | $R^1$ | $R^2$ | W | Q | Phys. Daten IR-Absorptionen [$cm^{-1}$] oder Fp. [°C] |
|---|---|---|---|---|---|
| 110 | $t-C_4H_9$ | H | S | 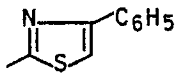 | 128-131 |
| 111 | $t-C_4H_9$ | H | O | 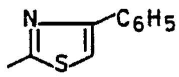 | |
| 112 | $CH_3$ | H | S | 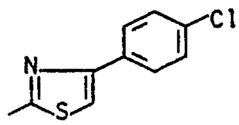 | |
| 113 | $i-C_3H_7$ | H | S | 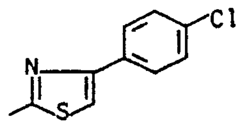 | |
| 114 | $t-C_4H_9$ | H | S | 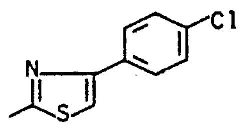 | |
| 115 | $t-C_4H_9$ | H | O | 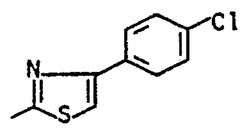 | |
| 116 | $t-C_4H_9$ | H | S | 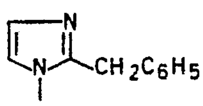 | |
| 117 | $t-C_4H_9$ | $CH_3$ | S | 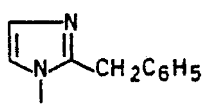 | |
| 118 | $t-C_4H_9$ | H | S | 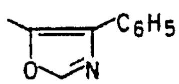 | |
| 119 | $t-C_4H_9$ | H | S | 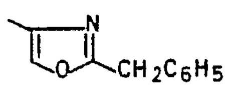 | |
| 120 | $t-C_4H_9$ | H | S | 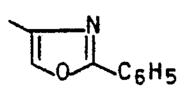 | |
| 121 | $t-C_4H_9$ | H | S | 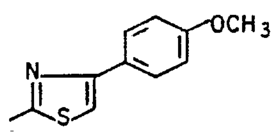 | |
| 122 | $t-C_4H_9$ | H | S | 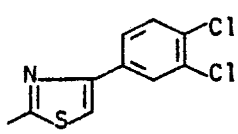 | |

| Nr. | $R^1$ | $R^2$ | W | Q | Phys. Daten IR-Absorptionen [$cm^{-1}$] oder Fp. [$^oC$] |
|---|---|---|---|---|---|
| 123 | $CH_3$ | H | S | 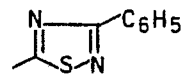 | |
| 124 | $i-C_3H_7$ | H | S | 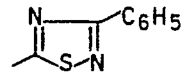 | |
| 125 | $t-C_4H_9$ | H | S | 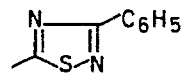 | |
| 126 | $t-C_4H_9$ | H | S | 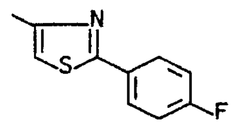 | 104-109 |

Anwendungsbeispiele

In den folgenden Beispielen wurden die erfindungsgemäßen Verbindungen bzw. sie enthaltende Mittel hinsichtlich ihrer Wirkung auf Tetranychus telarius, Caenorhabditis elegans, Plutella maculipennis und Dysdercus intermedius untersucht.

Die Konzentrationen, bei denen die Verbindungen eine 100 %ige Mortalität bzw. Hemmung bewirken, sind die jeweiligen Minimalkonzentrationen. Bei jeder Konzentration wurden mindestens 2 Versuche durchgeführt.

Die Reinheit der Wirkstoffe liegt >95 %. Als Formulierung wurde eine 0,1 %ige acetonische Lösung oder ein 10 gew.-%iges wäßriges Emulsionskonzentrat gewählt, das durch Emulgieren des Wirkstoffes in einer Mischung aus 70 Gew.-% Cyclohexanon, 20 Gew.-% Nekanil LN® ($\hat{=}$ Lutensol AP6, Netzmittel mit Emulgier- und Dispergierwirkung auf Basis ethoxylierter Alkylphenole) und 10 Gew.% Emulphor EL® ($\hat{=}$ Emulan EL®), Emulgator auf Basis ethoxylierter Fettalkohole) erhalten wurde. Die in den Beispielen angegebenen Konzentrationen wurden durch Verdünnen des formulierten Wirkstoffes mit Wasser erhalten.

Beispiel A

Tetranychus telarius (Rote Spinne)

Getopfte Buschbohnen, die das zweite Folgeblattpaar zeigen, werden in der Spritzkabine mit der wäßrigen Wirkstoffaufbereitung tropfnaß gespritzt. Die Pflanzen kommen dazu auf den Drehteller und werden von allen Seiten mit insgesamt 50 ml Spritzbrühe besprüht. Die Pflanzen müssen einen starken Milbenbefall und reichliche Eiablage aufweisen.

Die Wirkung wird nach 5 Tagen mittels Binokular bonitiert. Dabei wird darauf geachtet, ob alle Stadien gleichmäßig erfaßt sind. Die Pflanzen stehen während dieser Zeit unter normalen Gewächshausbedingungen.

In diesem Versuch wurde mit den Verbindungen 34 und 101 eine gute Wirkung erzielt.

Beispiel B

Kontaktwirkung auf Caenorhabditis elegans (Nematoden)

Auf die Oberfläche eines Nährbodens (5 ml in Kunststoffpetrischälchen ø 25 mm, Höhe 10 mm) gibt man 0,5 ml der 0,1 %igen acetonischen Wirkstofflösung. Nach Verdunsten des Acetons infiziert man mit 30 ml E-Coli-Bakterien und 50 ml Nematodensuspension.

Nach 48 Stunden ermittelt man die Kontaktwirkung in % Mortalität. Dabei zeigten die Verbindungen 34, 35 und 101 eine gute Wirkung.

Beispiel C

Kontaktwirkung auf Plutella maculipennis (Kohlschaben-Raupe)

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt. Nach 48 Stunden beurteilt man die Mortalität.

In diesem Versuch zeigten die Verbindungen 20, 35 und 83 eine gute Wirkung.

Beispiel D

Ovizide Wirkung auf Dysdercus intermedius

Stecketiketten werden am oberen Rand mit Klebestreifenstücken (ca. 0,8 cm) beklebt.

24 Stunden vor Versuchsbeginn werden die Eier durch Eintauchen in das "Sammelgefäß" an dem Klebestreifenrand befestigt. Die Eier werden für ca. 5 Sekunden in die wäßrige Wirkstoffaufbereitung getaucht, anschließend läßt man auf Filterpapier abtropfen.

Die zurechtgeschnittenen Etiketten werden in Plastikpaletten gestellt, Klebestreifen nach oben. Eine halbe mit Wasser durchfeuchtete Watterolle wird in den Becher gelegt, um Austrocknung zu vermeiden und die Palette mit einer Glasplatte abgedeckt. Nach ca. 8 Tagen wird bonitiert. (Bei der Kontrolle müssen die Larven geschlüpft sein.)

In diesem Versuch zeigten die Verbindungen 14, 21 und 34 eine gute Wirkung.

## Ansprüche

1. 3(2H)-Pyridazinonderivate der allgemeinen Formel I

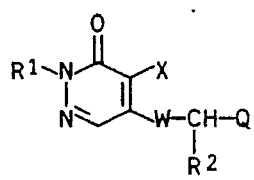

(I)

in der die Substituenten die folgende Bedeutung haben:
$R^1$ $C_1$-$C_8$-Alkyl,
$R^2$ Wasserstoff oder $C_1$-$C_4$-Alkyl,
X Halogen,
W Sauerstoff oder Schwefel und
Q einen 5- oder 6-gliedrigen Heterocyclus mit 2 bis 4 Heteroatomen aus der Gruppe Stickstoff, Sauerstoff oder Schwefel, der unsubstituiert oder ein- bis dreifach substituiert ist mit Halogen, $C_1$-$C_8$-Alkyl, $C_2$-$C_6$-Alkenyl, $C_2$-$C_4$-Alkinyl, $C_1$-$C_4$-Halogenalkyl, $C_1$-$C_8$-Alkoxy, $C_2$-$C_8$-Alkoxyalkyl, $C_3$-$C_8$-Cycloalkyl, Cyano, Nitro, Aryl und $C_7$-$C_{20}$-Aralkyl, welche unsubstiuiert, oder ein- bis dreifach durch Halogen, $C_1$-$C_8$-Alkyl, $C_1$-$C_8$-Alkoxy, $C_1$-$C_4$-Halogenalkyl, $C_1$-$C_4$-Halogenalkoxy, Cyano oder Nitro substituiert sind,
mit der Maßgabe, daß Q keinen Isoxazolyl- oder Pyridazinylrest bedeutet,
sowie pflanzenverträgliche Salze der 3(2H)-Pyridazinonderivate I.

2. Verfahren zur Herstellung von 3(2H)-Pyridazinonderivaten der Formel I gemäß Anspruch 1, dadurch

gekennzeichnet, daß man in an sich bekannter Weise 3(2H)-Pyridazinone der allgemeinen Formel II

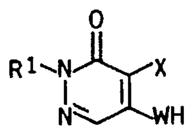

(II)

mit einer Verbindung der allgemeinen Formel III

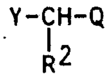

(III)

in der $R^2$ und Q die oben genannten Bedeutungen haben und Y für eine Abgangsgruppe steht, in Gegenwart einer Base umsetzt.

3. Schädlingsbekämpfungsmittel, enthaltend ein 3(2H)-Pyridazinonderivat gemäß Anspruch 1 neben hierfür üblichen Trägerstoffen.

4. Schädlingsbekämpfungsmittel nach Anspruch 3, dadurch gekennzeichnet, daß es 0,1 bis 95 Gew.% eines 3(2H)-Pyridazinonderivates der Formel I enthält.

5. Verwendung von 3(2H)-Pyridazinonderivaten I gemäß Anspruch 1 zur Bekämpfung von Schädlingen.

6. Verwendung von 3(2H)-Pyridazinonderivaten I

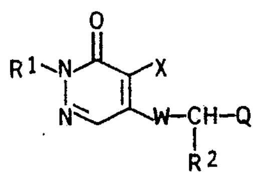

(I)

in der die Substituenten die folgende Bedeutung haben:
$R^1$ $C_1$-$C_8$-Alkyl,
$R^2$ Wasserstoff oder $C_1$-$C_4$-Alkyl,
X Halogen,
W Sauerstoff oder Schwefel und
Q einen 5- oder 6-gliedrigen Heterocyclus mit 2 bis 4 Heteroatomen aus der Gruppe Stickstoff, Sauerstoff oder Schwefel, der unsubstituiert oder ein- bis dreifach substituiert ist mit Halogen, $C_1$-$C_8$-Alkyl, $C_2$-$C_6$-Alkenyl, $C_2$-$C_4$-Alkinyl, $C_1$-$C_4$-Halogenalkyl, $C_1$-$C_8$-Alkoxy, $C_2$-$C_8$-Alkoxyalkyl, $C_3$-$C_8$-Cycloalkyl, Cyano, Nitro, Aryl und $C_7$-$C_{20}$-Aralkyl, welche unsubstiuiert, oder ein- bis dreifach durch Halogen, $C_1$-$C_8$-Alkyl, $C_1$-$C_8$-Alkoxy, $C_1$-$C_4$-Halogenalkyl, $C_1$-$C_4$-Halogenalkoxy, Cyano oder Nitro substituiert sind,
sowie pflanzenverträgliche Salze der 3(2H)-Pyridazinonderivate I zur Herstellung von Schneckenbekämpfungsmitteln.

7. Verfahren zur Herstellung molluskizider Mittel, dadurch gekennzeichnet, daß man 3(2H)-Pyridazinonderivate I gemäß Anspruch 6 mit inerten Verdünnungsmitteln und/oder oberflächenaktiven Stoffen vermischt.

8. Schneckenfraßköder, gekennzeichnet durch den Gehalt an 3(2H)-Pyridazinonderivaten I gemäß Anspruch 6.

9. Saatgutbeizmittel, gekennzeichnet durch den Gehalt an 3(2H)-Pyridazinonderivaten I gemäß Anspruch 6.

10. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines 3(2H)-Pyridazinonderivates der allgemeinen Formel I gemäß Anspruch 1 behandelt.